# EUROPÄISCHE PATENTSCHRIFT

(11) **EP 1 792 949 B1**
(45) Veröffentlichungstag und Bekanntmachung des Hinweises auf die Patenterteilung: **08.09.2010**
(21) Anmeldenummer: 06025134.5
(22) Anmeldetag: 05.12.2006
(51) Int. Cl.: C09B 23/00, C09B 23/08, C09B 23/06, C09B 23/02, G01N 33/533, G01N 33/58

(54) **Hydrophile Marker auf der Basis von diastereomeren Cyaninen**
Hydrophilic marker on the basis of diastereomeric cyanines
Marqueurs hydrophiles à base de cyanines diastereomeriques

(30) Priorität: 05.12.2005 DE 102005058587
(43) Veröffentlichungstag der Anmeldung: 06.06.2007
(73) Patentinhaber: Dyomics GmbH, 07745 Jena (DE)
(72) Erfinder: Czerney, Peter, Dr., 99425 Weimar (DE); Lehmann, Frank, Dr., 07749 Jena (DE); Wenzel, Matthias, Dr., 07749 Jena (DE); Frank, Wilhelm, Dr., 07743 Jena (DE); Schweder, Bernd, Dr., 07743 Jena (DE)
(74) Vertreter: Popp, Eugen

(56) Entgegenhaltungen:
- WO-A-02/26891
- WO-A-2004/039894
- WO-A-2006/111726
- DE-A1- 4 445 065
- JP-A- 4 186 342
- JP-A- 5 313 304
- A.MISHRA ET AL.: "Cyanines during the 1990s: A Review" CHEM.REV., Bd. 100, 2000, Seiten 1973-2011, XP002466001

## Beschreibung

Die Erfindung betrifft als Marker geeignete, stark hydrophile Verbindungen auf der Basis von diastereomeren Cyaninen und ihre Verwendung in optischen, insbesondere fluoreszenzoptischen Bestimmungs- und Nachweisverfahren.

Typische Verfahrensanwendungen beruhen auf der Reaktion von farbstoffmarkierten Biomolekülen, wie zum Beispiel Antigenen, Antikörpern oder DNA-Segmenten mit der jeweils komplementären Spezies. Damit werden unter anderem Messungen von Enzymkinetiken, Rezeptor-Ligand-Interaktionen und Nucleinsäure-Hybridisierungskinetiken *in vitro* als auch *in vivo* ermöglicht. Desweiteren sind die beanspruchten Marker für die pharmakologische Charakterisierung von Rezeptoren oder Wirkstoffen interessant.

Einsatzmöglichkeiten ergeben sich dadurch beispielsweise in der Medizin und Pharmazie, in der Bio- und Materialwissenschaft, bei der Umweltkontrolle und dem Nachweis von in der Natur und Technik vorkommenden organischen und anorganischen Mikroproben sowie anderes mehr.

Für diese Anwendungen werden üblicherweise Xanthylium-Salze (US 5,846,737; Molecular Probes) oder Cyanine, wie sie beispielsweise in der US-Patentschrift 5,627,027 beansprucht werden, verwendet.

Diese Marker haben den Nachteil, dass sie aufgrund der Planarität des π-Elektronensystems zur Aggregation und Dimerenbildung, insbesondere in wässrigen Systemen neigen. Desweiteren gehen ungenügend hydrophile Marker nicht spezifische Wechselwirkungen mit unterschiedlichen Oberflächen ein, was zu Problemen bei der Aufreinigung der entsprechenden Konjugate und zu einem nicht befriedigenden Signal/Rausch-Verhältnis führt.

In der jüngeren Vergangenheit hat es nicht an Versuchen gefehlt, durch die Einführung zusätzlicher, die Hydrophilie der Marker steigernde Substituenten die bekannten Nachteile der bisher bekannten Cyanine weiter zu verringern. Das geschah hauptsächlich durch die Einführung von zusätzlichen Sulfonsäure-Funktionen in das Cyanin-Chromophor.

In US 6,083,485 (Licha) bzw. US 09/969'853 und US 09/968'401 (Molecular Probes) werden Cyanine beschrieben, bei denen einer der am terminalen Indol-Heterocylus in 3-Position üblichen Methyl-Substituenten durch eine ω-Carboxyalkyl-Funktion substituiert ist und die bisherigen (z.B. im Cy3 oder Cy5) *N*-Alkyl- oder *N*-ω-Carboxyalkyl-Funktionen durch *N*-ω-Sulfonsäurealkyl-Funktionen ersetzt wurden.

In WO 05/044923 A1 (Amersham) werden Cyanine beschrieben, bei denen der am terminalen Indol-Heterocyclus in 3-Position übliche Methyl-Substituent durch eine ω-Sulfonsäurealkyl-Funktion substituiert ist. Ferner beschreibt die WO 2004/039894 A2 (Carnegie) chirale Indolzwischenstufen und herkömmliche fluoreszierende Cyaninfarbstoffe.

Die in diesen Patenten beschriebenen Cyanine mit mehr als zwei Sulfonsäure-Funktionen weisen gegenüber den in US 5,627,027 beschriebenen Cyaninen (Cy3, Cy5) eine erhöhte Löslichkeit und damit verbunden eine geringere Tendenz zur Dimerenbildung auf.

Wir fanden nun, dass es von besonderem Vorteil ist, in ihrem Substituentenmuster bisher noch nicht bekannte Cyanine zu verwenden, bei denen der eine terminale Indol-Heterocyclus in 3-Position mit einer solubilisierenden bzw. ionisierenden Gruppe (z. B. ω-Sulfonsäurealkyl-Funktion) und der andere terminale Heterocyclus mit einer Funktion zum Koppeln an Biomoleküle (z. B. einer ω-Carboxyalkyl-Funktion) ausgestattet ist.

Durch die Derivatisierung der beiden terminalen Indol-Heterocyclen in 3-Position, einmal mit einer ω-Sulfonsäurealkyl-Funktion und einmal mit einer ω-Carboxyalkyl-Funktion, weisen die hier beschriebenen Cyanine jeweils in 3-Position chirale Kohlenstoffatome auf, was bedeutet, dass hier ein Gemisch aus Diastereomeren vorliegt.

Die Vorteile der von uns an dieser Stelle erstmalig beschriebenen diastereomeren Cyanine liegen darin, dass bedingt durch die unterschiedliche räumliche Anordnung der Substituenten am Cyanin-Chromophor eine weitere Minimierung der Aggregation zwischen den Farbstoffmolekülen zu beobachten ist. Bedingt durch dieses spezielle Substituentenmuster, gekoppelt mit der starken Hydrophilie der neuen Cyanine (mindestens drei Sulfonsäurereste) wird das Ausfällen von Cyanin-Protein-Konjugaten mit hohem Cyanin-Protein-Verhältnis aus wässriger Lösung verhindert und unerwünschte unspezifische Wechselwirkungen gegenüber den im Stand der Technik bekannten Cyaninen werden deutlich vermindert. Gegenstand der Erfindung sind nun neue, speziell substituierte und stark hydrophile Marker auf der Basis von diastereomeren Cyaninen der allgemeinen Formeln **I** und **II,** 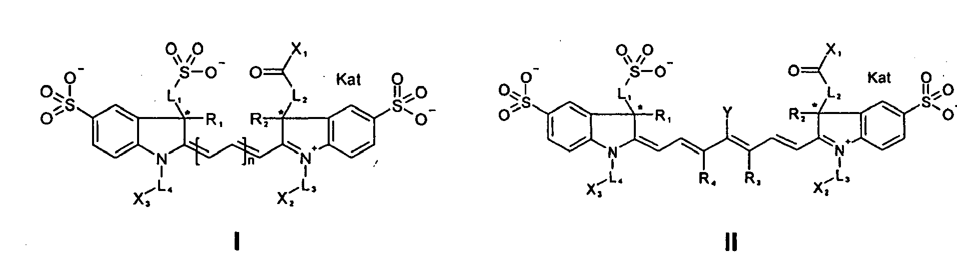 wobei es auch möglich ist, die Diastereomeren-Gemische, wie am Beispiel der allgemeinen Formel **I** gezeigt, zu trennen und dann die Enantiomeren-Gemische für optische Detektionsmethoden zu verwenden.

Diastereomer 1 (Enantiomeren-Gemisch)

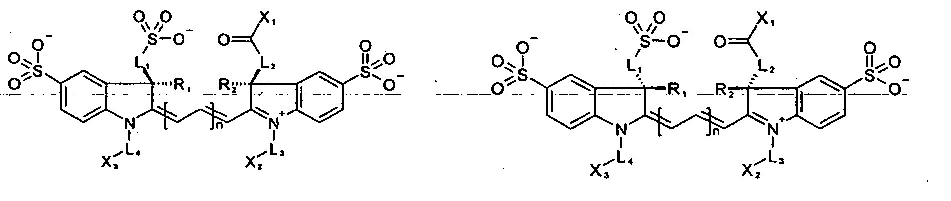

Diastereomer 2 (Enantiomeren-Gemisch) 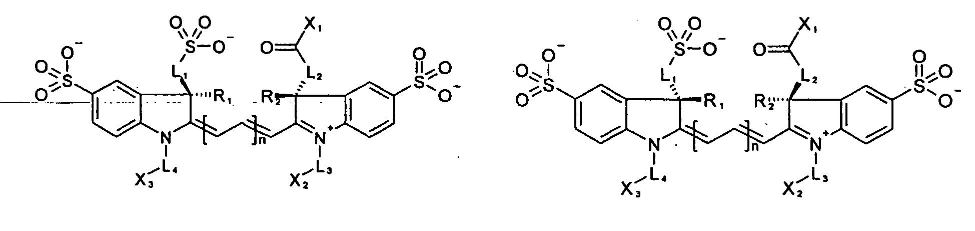 wobei
◆ R₁ und R₂ gleich oder unterschiedlich, jeweils eine aliphatisch-(vorzugsweise CH₃-) oder heteroaliphatische Gruppe sein können,
◆ L₁ - L₄ gleich oder unterschiedlich, jeweils eine divalente lineare (-(CH₂)ₒ- mit o = 1 - 15), gekreuzte oder cyclische Alkylen-Gruppe, die durch eine oder mehrere Heteroatome wie Sauerstoff, substituierter Stickstoff oder Schwefel substituiert sein kann, darstellen,
◆ X₁ gleich -OH, -SH, NH₂, -NH-NH₂, -F, -Cl, -Br, -I, -O-NHS (Hydroxy-Succinimidyl/Hydroxy-Sulfo-Succinimidyl), -O-STP (4-Sulfo-2,3,5,6-tetrafluorophenoxy), -O-TFP (2,3,5,6-tetrafluorophenoxy), Benzotriazol bzw. -NR-L-OH, -NR-L-O-Phosphoramidit, -NR-L-SH, -NR-L-CO-NH₂,-NR-L-CO-NH-NH₂, -NR-L-CHO, -NR-L-COOH, -NR-L-CO-ONHS, -NR-L-CO-O-STP, -NR-L-CO-O-TFP, -NR-L-NH-CO-CH₂-I und -NR-L-Maleimid, wobei L eine divalente lineare (-(CH₂)_{o'}- mit o' = 1 - 15), gekreuzte oder cyclische Alkylen-Gruppe darstellt, die durch ein oder mehrere Heteroatome wie Sauerstoff, substituierter Stickstoff oder Schwefel substituiert und R gleich Wasserstoff oder R₁ oder R₂ sein kann,
◆ X₂ und X₃ gleich oder unterschiedlich Wasserstoff, Alkyl-, *tert*-Alkyl-, Aryl-, Carboxyaryl-, Dicarboxyaryl-, Heteroaryl-, Cycloalkyl-, Heterocycloalkyl-, Alkyloxy-, Alkylmercapto-, Aryloxy-, Arylmercapto-, Hydroxy-, Amino-, Nitro-, Cyano-Reste sein können bzw. einen solubilisierenden bzw. ionisierbaren bzw. ionisierten Substituenten wie -SO₃⁻, -PO₃²⁻, -CO₂⁻, *tert-*Ammonium, Cyclodextrin oder Zucker darstellen,
◆ Y für ein Halogenatom wie Fluor, Chlor oder Brom, eine substituierte Phenoxy- oder eine substituierte Arylmercapto- (Phenyl-sulfanyl-) Funktion steht,
◆ Kat für eine bestimmte Anzahl von Na⁺, K⁺, Ca²⁺, Ammonium oder andere Kationen steht, die zum Ausgleich der durch das Cyanin **I** bzw. **II** vorgegebenen negativen Ladung notwendig sind,
◆ für n im Cyanin **I** der Wert von 0 - 3 stehen kann und
◆ R₃ und R₄ in **II** gleich oder unterschiedlich, jeweils eine aliphatisch-(vorzugsweise CH₃-) oder heteroaliphatische Gruppe sein kann bzw. zusammen das divalente Strukturelement -(CH₂)ₘ-, -(CH₂)ₘO(CH₂)_{m'}-,
   -(CH₂)ₘS(CH₂)_{m'}-, -(CH₂)ₘCH=CH- oder -OCH=CH- bilden und m bzw. m' gleich oder unterschiedlich die Werte von 2-6 darstellen können,

Die erfindungsgemäßen Cyanine können als Chromophore/Fluorophore zur optischen Markierung und damit dem qualitativen und/oder quantitativen Nachweis von Proteinen, Nukleinsäuren, Oligomeren, DNA, RNA, biologischen Zellen, Lipiden, Mono-, Oligo- und Polysacchariden, Liganden, Rezeptoren, Polymeren, Pharmaka oder Polymerpartikeln verwendet werden.
Die kovalente Kopplung der neuen Cyanine erfolgt dabei über die in 3-Stellung am Indol-Ring stehende ω-Carboxyalkyl-Funktion, die beispielsweise nach bekannten Verfahren aus der Proteinchemie als NHS-Ester, Säurefluorid oder STP-Ester aktiviert werden kann und mit der Aminfunktion des Biomoleküls unter Ausbildung einer Säureamidfunktion umgesetzt wird.
Diese Kopplungsreaktion wird vorteilhaft in organischen oder wäßrigen Lösungen durchgeführt. Die aus den erfindungsgemäßen Cyaninen und Biomolekülen bestehenden Konjugate weisen fluoreszierende Eigenschaften auf.

Die erfindungsgemäßen Cyanine finden in optischen, insbesondere fluoreszenzoptischen qualitativen und quantitativen Bestimmungsverfahren einschließlich Immuntests, Hybridisierungsverfahren, chromatographischen oder elektrophoretischen Verfahren, FRET-Systemen und Hoch-Durchsatz-Screenings oder zur Analyse von Rezeptor-Liganden-Wechselwirkungen auf einem Mikroarray Verwendung.

Die Cyanine der allgemeinen Formeln **I** und/oder **II** können als Farbstoffe zur optischen Markierung von organischen oder anorganischen Erkennungseinheiten, zum Beispiel von Aminosäuren, Peptiden, Proteinen, Antigenen, Haptenen, Enzymsubstraten, Enzym-Cofaktoren, Biotin, Carotinoiden, Hormonen, Neurohormonen, Neurotransmittern, Wachstumsfaktoren, Lektinen, Toxinen, Kohlenhydraten, Oligosacchariden, Polysacchariden, Dextranen, Nukleinsäuren, Oligonukleotiden, DNA, RNA, biologischen Zellen, Lipiden, rezeptorbindenden Pharmaka oder organischen bzw. anorganischen polymeren Trägermaterialien verwendet werden.

Die Markierung der Erkennungseinheiten kann dabei auch durch die Ausbildung von ionischen Wechselwirkungen zwischen den Cyaninen der allgemeinen Formeln **I** und/oder **II** und den zu markierenden Materialien erfolgen.

Weiterhin besteht auch die Möglichkeit, die Erkennungseinheit oder das Trägermaterial kovalent mit dem Cyanin zu verbinden. Diese Kopplungsreaktion kann in wäßriger oder überwiegend wäßriger Lösung und vorzugsweise bei Raumtemperatur durchgeführt werden. Dabei entsteht ein Konjugat zur qualitativen oder quantitativen Bestimmung von unterschiedlichen Biomaterialien bzw. anderen organischen und anorganischen Materialien unter Benutzung optischer Verfahren.

Sowohl die Cyanine der allgemeinen Formeln **I** und/oder **II** und davon abgeleitete Systeme können in optischen, insbesondere fluoreszenzoptischen, qualitativen und quantitativen Bestimmungsverfahren zur Diagnostik von Zelleigenschaften (Molekulare Bildgebung), in Biosensoren (*point of care*-Messungen), zur Erforschung des Genoms und in Miniaturisierungstechnologien eingesetzt werden. Typische Anwendungen erfolgen in der Zytometrie und Zellsortierung, der Fluoreszenz-Korrelations-Spektroskopie (FCS), im *Ultra-High-Throughput-Screening* (UHTS), bei der *multicolor* Fluoreszenz-*in-situ*-Hybridisierung (FISH), in FRET-Systemen und in Mikroarrays (DNA- und Protein-Chips).

Dabei ist ein Mikroarray eine rasterartige Anordnung von auf mindestens einer Oberfläche immobilisierten Molekülen, die zum Studium von Rezeptor-Liganden-Wechselwirkungen verwendet werden kann. Eine rasterartige Anordnung bedeutet mehr als zwei voneinander verschiedene Moleküle, welche sich innerhalb einer Fläche befinden und dort in unterschiedlichen, vorher definierten Regionen mit bekannter Position immobilisiert sind.

Ein Rezeptor ist ein Molekül, das eine Affinität zu einem gegebenen Liganden besitzt. Rezeptoren können natürlich vorkommende oder künstlich hergestellte Moleküle sein. Rezeptoren können in reiner Form oder gebunden an andere Spezies eingesetzt werden. Rezeptoren können kovalent oder nichtkovalent entweder direkt oder durch bestimmte Kopplungsvermittler an einen Bindungspartner angeknüpft werden.

Beispiele für Rezeptoren, die durch diese Erfindung detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Zellen, Zellfragmente, Gewebefragmente, Proteine und Antikörper ein, sind aber nicht auf die angeführten Stoffe beschränkt.

Ein Ligand ist ein Molekül, das von einem bestimmten Rezeptor erkannt wird. Beispiele für Liganden, die durch die erfindungsgemäßen Verbindungen detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Proteine und Antikörper ein, sind aber nicht auf die angeführten Stoffe beschränkt.

Die erfindungsgemäßen Cyanine sind relativ einfach durch das Kondensieren der beiden unterschiedlich substituierten CH-aciden Indol-Heterocyclen und eines *C*-1, *C*-3 oder *C*-5 Bausteins herzustellen.

Weitere Verfahren zu ihrer Herstellung bestehen darin, dass in einer 1. Reaktionsstufe einer der *CH*-aciden Indol-Heterocyclen mit dem *C*-1, *C*-3 oder *C*-5 Baustein kondensiert wird und nach Isolierung des 1:1-Kondensationsproduktes in einer nachfolgenden Kondensation mit dem zweiten *CH*-aciden Indol-Heterocyclus zum Cyanin umgesetzt wird. Dabei ist die Reihenfolge der Verwendung der CH-aciden Indol-Heterocyclen unerheblich. Dadurch ist in wenigen Reaktionsschritten eine Vielzahl unterschiedlich funktionalisierter, stark hydrophiler, diastereomerer Cyanine, die sich hinsichtlich der Gesamtladung und der Spezifität/Reaktivität der zur Immobilisierung genutzten aktivierten Gruppen unterscheiden, auf einfache Weise herstellbar.

Die Erfindung soll nachstehend anhand der Ausführungsbeispiele 1 und 2 näher erläutert werden.

**Ausführungsbeispiel 1** (Verbindungen der allgemeinen Formel **I**)

### 1. Synthese von 1-Ethyl-3-methyl-2-((E)-2-phenylamino-vinyl)-5-sulfo-3-(3-sulfopropyl)-3H-indolium Natriumsalz

0,794 g (2 mmol) 3-(3-Sulfopropyl)-2,3-dimethyl-5-sulfonato-1-ethyl-3*H*-indolium Natriumsalz und 0,49 g (2,5 mmol) N,N'-Diphenyl-formamidin werden in 20 ml Methanol aufgenommen und ca. 4 h am Rückfluß gerührt. Nach dem Abkühlen auf RT wird das Lösungsmittelgemisch im Vakuum abdestilliert. Der Rückstand wird gut mit Essigester gewaschen. Man erhält einen dunkelgelben Feststoff, der ohne weitere Reinigung weiter umgesetzt werden kann.

### 2. 2-{(E)-3-[3-(3-Carboxy-propyl)-3-methyl-5-sulfo-1-(3-sulfo-propyl)-1,3-dihyro-indol-(2E)-ylidene]-propenyl}-1-ethyl-3-methyl-5-sulfo-3-(3-sulfo-propyl)-3H-indolium Trinatriumsalz

500 mg (1 mmol) 1-Ethyl-3-methyl-2-((E)-2-phenylamino-vinyl)-5-sulfo-3-(3-sulfopropyl)-3*H*-indolium Natriumsalz und 483 mg (1 mmol) 3-(3-Carboxypropyl)-2,3-dimethyl-5-sulfonato-1-(3-sulfopropyl)-3*H*-indolium Natriumsalz werden in einem Gemisch von 10 ml Essigsäure und 10 ml Acetanhydrid gelöst. Nun werden 5 ml Pyridin zugegeben. Die Lösung wird ca. 15 min am Rückfluß gerührt.
Nachdem auf RT abgekühlt wurde, gibt man ca. 20 ml Ether zu. Der entstandene Niederschlag wird abgesaugt, mit Ether gewaschen und getrocknet.
Der Rückstand wird säulenchromatografisch gereinigt: RP-18: Acetonitril/ Wasser, 2/8 + 1% conc. HCl; Hierbei werden die Diastereomeren voneinander getrennt. Die guten Fraktionen werden, getrennt nach den Diastereomeren, vereinigt, mit NaHCO₃ neutralisiert und eingedampft. Die Trennung wird über eine RP-18-Säule - Acetonitril/Wasser, 2/8 - ohne Säure wiederholt.

Die entsprechenden Fraktionen werden vereinigt und das Lösungsmittel wird abdestilliert. Die beiden Produkte (Diastereomeren) werden im Hochvakuum getrocknet.
Die Ausbeute liegt bei ca. 15 % pro Diastereomer.
Diastereomer 1:
UV-vis(Ethanol): λₘₐₓ = 560 nm
MS (ES,-) [M/z]: 271.8 [M]³⁻
Diastereomer 2:
UV-vis(Ethanol): λₘₐₓ = 562 nm
MS (ES,-) [M/z]: 419.1 [M + Na]²⁻; 271.8 [M]³⁻

### 3. Synthese von 1-Ethyl-3-methyl-2-((1E,3E)-4-phenylamino-buta-1,3-dienyl)-5-sulfo-3-(3-sulfo-propyl)-3H-indolium Natriumsalz

0,794 g (2 mmol) 3-(3-Sulfopropyl)-2,3-dirnethyl-5-sulfonato-1-ethyl-3*H*-indolium Natriumsalz und 0,65 g (2,5 mmol) Malonaldehyd-bisphenylimin-hydrochlorid werden in einem Gemisch aus 10 ml Essigsäure und 10 ml Acetanhydrid gelöst und ca. 4 h bei 120 °C gerührt. Nach dem Abkühlen auf RT wird das Lösungsmittelgemisch im Vakuum abdestilliert. Der Rückstand wird mit Essigester gewaschen. Man erhält einen dunkelbraunen Feststoff, der ohne weitere Reinigung umgesetzt werden kann.

### 4. Synthese von 2-{(1E,3E)-5-[3-(3-Carboxy-propyl)-3-methyl-5-sulfo-1-(3-sulfopropyl)-1,3-dihydro-indol-(2E)-ylidene]-penta-1,3-dienyl}-1-ethyl-3-methyl-5-sulfo-3-(3-sulfo-propyl)-3H-indolium Trinatriumsalz

516 mg (1 mmol) 1-Ethyl-3-methyl-2-((1E,3E)-4-phenylamino-buta-1,3-dienyl)-5-sulfo-3-(3-sulfo-propyl)-3*H*-indolium Natriumsalz und 483 mg (1 mmol) 3-(3-Carboxypropyl)-2,3-dimethyl-5-sulfonato-1-(3-sulfopropyl)-3*H*-indolium Natriumsalz werden in einem Gemisch von 10 ml Essigsäure und 10 ml Acetanhydrid gelöst. Nun werden 5 ml Pyridin zugegeben. Die Lösung wird ca. 15 min am Rückfluß gerührt. Nachdem auf RT abgekühlt wurde, gibt man ca. 20 ml Ether zu. Der entstandene Niederschlag wird abgesaugt, mit Ether gewaschen und getrocknet.
Der Rückstand wird säulenchromatografisch gereinigt: RP-18 Acetonitril/ Wasser, 2/8 + 1 % conc. HCl; Hierbei werden die Diastereomeren voneinander getrennt. Die guten Fraktionen werden, getrennt nach den Diastereomeren, vereinigt, neutralisiert und eingedampft. Die Trennung wird über eine RP-18-Säule - Acetonitril/ Wasser, 2/8 - ohne Säure wiederholt.
Die entsprechenden Fraktionen werden vereinigt und das Lösungsmittel wird abdestilliert. Die beiden Produkte (Diastereomeren) werden im Hochvakuum getrocknet.
Die Ausbeute liegt bei ca. 15 % pro Diastereomer.
Diastereomer 1:
UV-vis(Ethanol): λₘₐₓ = 655 nm
MS (ES,-) [M/z]: 432.2 [M + Na]²⁻; 280.5 [M]³⁻
Diastereomer 2:
UV-vis(Ethanol): λₘₐₓ = 654 nm
MS (ES,-) [M/z]: 432.1 [M + Na]²⁻; 280.5 [M]³⁻

### 5. Synthese von 3-Methyl-2-((1E,3E)-4-phenylamino-buta-1,3-dienyl)-5-sulfo-1,3-bis-(3-sulfo-propyl)-3H-indolium Bisnatriumsalz

1,03 g (2 mmol) 1,3-bis-(3-Sulfopropyl)-2,3-dimethyl-5-sulfonato-3H-indolium Bisnatriumsalz und 0,65 g (2,5 mmol) Malonaldehyd-bisphenylimin-hydrochlorid werden in einem Gemisch aus 10 ml Essigsäure und 10 ml Acetanhydrid gelöst und ca. 4 h bei 120 °C gerührt. Nach dem Abkühlen auf RT wird das Lösungsmittelgemisch im Vakuum abdestilliert. Der Rückstand wird mit Essigester gewaschen. Man erhält einen dunkelbraunen Feststoff, der ohne weitere Reinigung umgesetzt werden kann.

### 6. Synthese von 2-{(1E,3E)-5-[3-(3-Carboxy-propyl)-3-methyl-5-sulfo-1-(3-sulfopropyl)-1,3-dihydro-indol-(2E)-ylidene]-penta-1,3-dienyl}-3-methyl-5-sulfo-1,3-bis-(3-sulfo-propyl)-3H-indolium Tetranatriumsalz

642 mg (1 mmol) 3-Methyl-2-((1E,3E)-4-phenylamino-buta-1,3-dienyl)-5-sulfo-1,3-bis-(3-sulfo-propyl)-3*H*-indolium Bisnatriumsalz und 483 mg (1 mmol) 3-(3-Carboxypropyl)-2,3-dimethyl-5-sulfonato-1-(3-sulfopropyl)-3*H*-indolium Natriumsalz werden in einem Gemisch von 10 ml Essigsäure und 10 ml Acetanhydrid gelöst. Nun werden 5 ml Pyridin zugegeben. Die Lösung wird ca. 15 min am Rückfluß gerührt.

Nachdem auf RT abgekühlt wurde, gibt man ca. 20 ml Ether zu. Der entstandene Niederschlag wird abgesaugt, mit Ether gewaschen und getrocknet.
Der Rückstand wird säulenchromatografisch gereinigt: RP-18 Acetonitril/ Wasser, 2/8 + 1% conc. HCl. Hierbei werden die Diastereomeren voneinander getrennt. Die guten Fraktionen werden getrennt nach den Diastereomeren vereinigt, mit NaHCO₃ neutralisiert und eingedampft. Die Trennung wird über eine RP-18-Säule - Acetonitril/Wasser, 2/8 - ohne Säure wiederholt.
Die entsprechenden Fraktionen werden vereinigt und das Lösungsmittel wird abdestilliert. Die beiden Produkte (Diastereomeren) werden im Hochvakuum getrocknet.
Die Ausbeute liegt bei ca. 10 % pro Diastereomer.
Diastereomer 1:
UV-vis(Ethanol): λₘₐₓ = 654 nm
MS (ES,-) [M/z]: 490.2 [M + 2Na]²⁻; 319,4 [M + Na]³⁻; 233.6 [M]⁴⁻
Diastereomer 2:
UV-vis(Ethanol): λₘₐₓ = 655 nm
MS (ES,-) [M/z]: 490.2 [M + 2Na]²⁻; 319,3 [M + Na]³⁻; 233.5 [M]⁴⁻

### 7. Synthese von 2-{(1E,3E,5E)-7-[3-(3-Carboxy-propyl)-3-methyl-5-sulfo-1-(3-sulfopropyl)-1,3-dihydro-indol-(2E)-ylidene]-hepta-1,3,5-trienyl}-1-ethyl-3-methyl-5-sulfo-3-(3-sulfo-propyl)-3H-indolium Trinatriumsalz

397 mg (1 mmol) 3-(3-Sulfopropyl)-2,3-dimethyl-5-sulfonato-1-ethyl-3*H*-indolium Natriumsalz und 284 mg (1 mmol) Phenyl-[(2E,4E)-5-phenylamino-penta-2,4-dien-(E)-ylidene]-ammoniumchlorid werden in 10 ml Essigsäure und 10 ml Acetanhydrid gelöst und 1 h bei 120 °C gerührt. Nun werden 483 mg (1 mmol) 3-(3-Carboxypropyl)-2,3-dimethyl-5-sulfonato-1-(3-sulfopropyl)-3*H*-indolium Natriumsalz und 5 ml Pyridin zugegeben. Das Gemisch wird nun bei 170 °C ca. 20 min gerührt.
Nach dem Abkühlen auf RT gibt man ca. 20 ml Ether zu. Der entstandene Niederschlag wird abgesaugt, mit Ether gewaschen und getrocknet.
Der Rückstand wird säulenchromatografisch gereinigt: RP-18 Acetonitril/ Wasser, 2/8 + 1% conc. HCl. Hierbei werden die Diastereomeren voneinander getrennt. Die guten Fraktionen werden getrennt nach den Diastereomeren vereinigt, mit NaHCO₃ neutralisiert und eingedampft. Die Trennung wird über eine RP-18-Säule - Acetonitril/Wasser, 2/8 - ohne Säure wiederholt.
Die entsprechenden Fraktionen werden vereinigt und das Lösungsmittel wird abdestilliert. Die beiden Produkte (Diastereomeren) werden im Hochvakuum getrocknet.
Die Ausbeute liegt bei ca. 2 % pro Diastereomer.
Diastereomer 1:
UV-vis(Ethanol): λₘₐₓ = 752 nm
MS (ES,-) [M/z]: 445.1 [M + Na]²⁻; 289.2 [M]³⁻
Diastereomer 2:
UV-vis(Ethanol): λₘₐₓ = 751 nm
MS (ES,-) [M/z]: 445.1 [M + Na]²⁻; 289.2 [M]³⁻

### Ausführungsbeispiel 2 - (Verbindungen der allgemeinen Formel II) 8. 2-((E)-2-{3-[2-[3-(3-Carboxy-propyl)-3-methyl-5-sulfo-1-(3-sulfo-propyl)-1,3-dihydro-indol-(2E)-ylidene]-eth-(E)-ylidene]-2-chloro-cyclohexyl}-vinyl)-1-ethyl-3-methyl-5-sulfo-3-(3-sulfo-propyl)-3H-indolium Trinatriumsalz

397 mg (1 mmol) 3-(3-Sulfopropyl)-2,3-dimethyl-5-sulfonato-1-ethyl-3*H*-indolium Natriumsalz und 358 mg (1 mmol) [1-{2-Chloro-3-[1-phenylamino-meth-(E)-ylidene]-cyclohex-1-enyl}-meth-(E)-ylidene]-phenyl-ammoniumchlorid werden in 10 ml Essigsäure und 10 ml Acetanhydrid gelöst und 1 h bei 120 °C gerührt. Nun werden 483 mg (1 mmol) 3-(3-Carboxypropyl)-2,3-dimethyl-5-sulfonato-1-(3-sulfopropyl)-3*H-*indolium Natriumsalz und 5 ml Pyridin zugegeben. Das Gemisch wird nun bei 170 °C ca. 20 min gerührt.
Nach dem Abkühlen auf RT gibt man ca. 20 ml Ether zu. Der entstandene Niederschlag wird abgesaugt, mit Ether gewaschen und getrocknet.
Der Rückstand wird säulenchromatografisch gereinigt: RP-18 Acetonitril/ Wasser, 2/8 + 1% conc. HCl. Hierbei werden die Diastereomeren voneinander getrennt. Die guten Fraktionen werden getrennt nach den Diastereomeren vereinigt, mit NaHCO₃ neutralisiert und eingedampft. Die Trennung wird über eine RP-18-Säule - Acetonitril/Wasser, 2/8 - ohne Säure wiederholt.

Die entsprechenden Fraktionen werden vereinigt und das Lösungsmittel wird abdestilliert. Die beiden Produkte (Diastereomeren) werden im Hochvakuum getrocknet.
Die Ausbeute liegt bei ca. 1 % pro Diastereomer.
Diastereomer 1:
UV-vis(Ethanol): λₘₐₓ = 784 nm
MS (ES,-) [M/z]: 482.1 [M + Na]²⁻; 313.7 [M]³⁻
Diastereomer 2:
UV-vis(Ethanol): λₘₐₓ = 784 nm
MS (ES,-) [M/z]: 482.2 [M + Na]²⁻; 313.7 [M]³⁻

### Allgemeine Vorschrift zur Synthese von N-Hydroxysuccinimid-Estern

20 µmol Farbstoff vom Typ **I** oder **II** mit X₁ = OH, 8 mg (40 µmol) Dicyclohexylcarbodiimid und 5 mg (40 µmol) N-Hydroxysuccinimid werden in 1 ml trockenem DMF gelöst. Dann gibt man 6 µl (40µmol) Triethylamin zu. Das Reaktionsgemisch wird 24 Stunden bei RT gerührt und anschließend filtriert. Im Anschluß daran wird das Lösungsmittel abgezogen und der Rückstand mit Diethylether gewaschen. Diese Reaktion verläuft quantitativ.

Allgemeine Vorschrift zur Synthese von Maleimid-Farbstoff-Konjugaten

20 µmol Farbstoff vom Typ **I** oder **II** mit X₁ = O-NHS werden in 2 ml trockenem DMF gelöst. Zu dieser Lösung gibt man 7,6 mg (30 µmol) 2-Maleimidoethylaminetrifluoracetat und danach 5 µl (30 µmol) N-Ethyldiisopropylamin. Das Reaktionsgemisch wird ca. 3 h bei RT gerührt. Im Anschluß daran wird das Lösungsmittel im Vakuum abdestilliert. Der Rückstand wird nun mit Ether und Aceton gewaschen. Diese Reaktion verläuft quantitativ.

Allgemeine Vorschrift zur Synthese von lodacetamid-Farbstoff-Konjugaten

20 µmol Farbstoff vom Typ **I** oder **II** mit X₁ = O-NHS werden in 2 ml trockenem DMF gelöst. Zu dieser Lösung gibt man 40 mg (300 µmol) Ethylendiamindihydrochlorid und danach 26 µl (150 µmol) N-Ethyldiisopropylamin. Das Reaktionsgemisch wird ca. 3 h bei RT gerührt. Im Anschluß daran wird das Lösungsmittel im Vakuum abdestilliert. Der Rückstand wird nun in Methanol aufgenommen und das überschüssige Ethylendiamindihydrochlorid abfiltriert. Das Methanol wird im Vakuum abdestilliert. Der Rückstand wird wiederum in 2 ml trockenem DMF gelöst und mit 7 mg (25 µmol) N-Succinimidyl Jodoacetat und 4 µl (25 µmol) N-Ethyldiisopropylamin versetzt. Das Reaktionsgemisch wird ca. 3 h bei RT gerührt. Das Lösungsmittel wird im Vakuum abdestilliert und der Rückstand wird mittels HPLC gereinigt.

## Patentansprüche

1. Hydrophile Marker auf Basis eines Gemischs diastereomerer Cyanine der allgemeinen Struktur I bzw. II 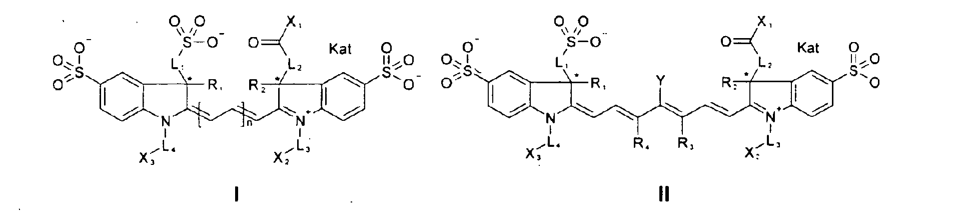 wobei
R₁ und R₂ gleich oder unterschiedlich, jeweils eine aliphatisch- (vorzugsweise CH₃-) oder heteroaliphatische Gruppe sein können,
L₁ - L₄ gleich oder unterschiedlich, jeweils eine divalente lineare (-(CH₂)ₒ- mit o = 1 - 15), gekreuzte oder cyclische Alkylen-Gruppe, die durch eine oder mehrere Heteroatome wie Sauerstoff, substituierter Stickstoff oder Schwefel substituiert sein kann, darstellen,
X₁ gleich -OH, -SH, NH₂, -NH-NH₂, -F, -Cl, -Br, -I, -O-NHS (Hydroxysuccinimidyl/Hydroxysulfo-Succinimidyl), -O-STP (4-Sulfo-2,3,5,6-tetrafluorophenoxy), -O-TFP (2,3,5,6-Tetrafluorophenoxy), Benzotriazol bzw. -NR-L-OH, -NR-L-O-Phosphoramidit, -NR-L-SH, -NR-L-CO-NH₂, -NR-L-CO-NH-NH₂, -NR-L-CHO, -NR-L-COOH, -NR-L-CO-ONHS, -NR-L-CO-O-STP, -NR-L-CO-O-TFP, -NR-L-NH-CO-CH₂-I und -NR-L-Maleimid, wobei L eine divalente lineare (-(CH₂)ₒ- mit o' = 1 - 15), gekreuzte oder cyclische Alkylen-Gruppe darstellt, die durch ein oder mehrere Heteroatome wie Sauerstoff, substituierter Stickstoff oder Schwefel substituiert und R gleich Wasserstoff oder R₁ oder R₂, sein kann,
X₂ und X₃ gleich oder unterschiedlich Wasserstoff, Alkyl-, *tert*-Alkyl-, Aryl-, Carboxyaryl-, Dicarboxyaryl-, Heteroaryl-, Cycloalykl-, Heterocycloalkyl-, Alkyloxy-, Alkylmercapto-, Aryloxy-, Arylmercapto-, Hydroxy-, Amino-, Nitro-, Cyano-Reste sein können bzw. einen solubilisierenden bzw. ionisierbaren bzw. ionisierten Substituenten wie -SO₃⁻, -PO₃²-, -CO₂⁻, *tert*-Ammonium, Cyclodextrin oder Zucker darstellen,
Y für ein Halogenatom wie Fluor, Chlor oder Brom, eine substituierte Phenoxy- oder eine substituierte Arylmercapto- (Phenyl-sulfanyl-) Funktion steht,
Kat für eine bestimmte Anzahl von Na⁺, K⁺, Ca²⁺, Ammonium oder andere Kationen steht, die zum Ausgleich der durch das Cyanin I bzw. II vorgegebenen negativen Ladung notwendig sind,
für n im Cyanin 1 der Wert von 0 - 3 stehen kann und
R₃ und R₄ in II gleich oder unterschiedlich, jeweils eine aliphatisch- (vorzugsweise CH₃-) oder heteroaliphatische Gruppe sein kann bzw. zusammen das divalente Strukturelement -(CH₂)ₘ-, -(CH₂)ₘO(CH₂)ₘ-, -(CH₂)ₘS(CH₂)ₘ-, -(CH₂)ₘCH=CH- oder -OCH=CH- bilden und m bzw. m' gleich oder unterschiedlich die Werte von 2 - 6 darstellen können.

2. Hydrophile Marker gemäß Anspruch 1, **dadurch gekennzeichnet, dass** die in 3-Stellung am Indol-Ring stehende ω-Carboxyalkyl-Funktion, beispielsweise als NHS-Ester, Säurefluorid, TFP- oder STP-Ester aktiviert vorliegt.

3. Hydrophile Marker gemäß Anspruch 1, **dadurch gekennzeichnet, dass** die in 3-Stellung am Indol-Ring stehende ω-Carboxyalkyl-Funktion genutzt wird, um über eine Säureamid-Funktion die jeweiligen Cyanine als Maleimide, Halogenacetamide, Hydrazide, Amine- oder Phosphoramidite zu aktivieren.

4. Hydrophile Marker gemäß den Ansprüchen 1 und 3, **dadurch gekennzeichnet, dass** anstelle der Diastereomeren-Gemische die entsprechenden reinen Diasteromere zum Markieren von Biomolekülen eingesetzt werden.

5. Hydrophile Marker gemäß den Ansprüchen 1 - 4, **dadurch gekennzeichnet, dass** diese zum Markieren von Biomolekülen eingesetzt werden.

6. Verwendung der hydrophilen Marker nach den Ansprüchen 1 - 4 als Cyanine zur kovalenten Anbindung von Aminosäuren, Proteinen, Antikörpern, Nucleinsäuren, Oligomeren, DNA, RNA, biologischen Zellen, Lipiden, Haptenen, Polymeren, Pharmaka oder Polymerpartikeln und Anwendung der Cyanin-Konjugate in optischen, insbesondere fluoreszenzoptischen qualitativen und quantitativen Bestimmungsverfahren einschließlich Immuntests, Hybridisierungsverfahren, FRET-Systemen, chromatographischer oder elektrophoretischer Verfahren und des Hoch-Durchsatz-Screenings.

## Claims

1. Hydrophilic markers based on a mixture of diastereoisomeric cyanines of the general structure I and/or II 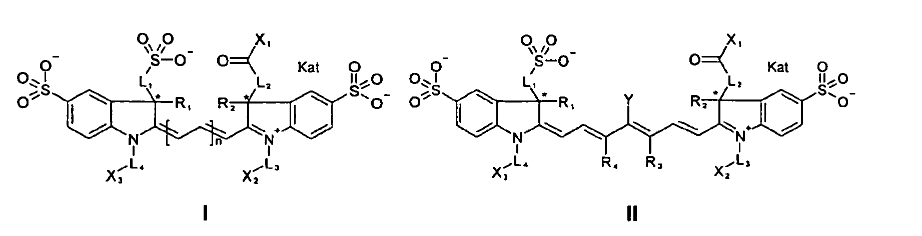 wherein
R₁ and R₂ are identical or different and may each be an aliphatic (preferably CH₃-) or heteroaliphatic group,
L₁ - L₄ are identical or different and are each a divalent linear (-(CH₂)ₒ wherein o = 1 - 15), branched or cyclic alkylene group which may be substituted by one or more hetero atoms such as oxygen, substituted nitrogen or sulphur,
X₁ is -OH, -SH, NH₂, -NH-NH₂, -F, -Cl, -Br, -I, -O-NHS (hydroxy-succinimidyl/hydroxysulpho-succinimidyl), -O-STP (4-sulpho-2,3,5,6-tetrafluorophenoxy), -O-TFP (2,3,5,6-tetrafluorophenoxy), benzotriazole or -NR-L-OH, -NR-L-O-phosphoramidite, -NR-L-SH, -NR-L-CO-NH₂, -NR-L-CO-NH-NH₂, -NR-L-CHO, -NR-L-COOH, -NR-L-CO-ONHS, -NR-L-CO-O-STP, -NR-L-CO-O-TFP,
-NR-L-NH-CO-CH₂-I and -NR-L-maleimide, wherein L is a divalent linear (-(CH₂)_{o'}- wherein o' = 1 - 15), branched or cyclic alkylene group which may be substituted by one or more hetero atoms such as oxygen, substituted nitrogen or sulphur, and R may be hydrogen or R₁ or R₂,
X₂ and X₃ are identical or different and may be hydrogen, alkyl, *tert*-alkyl, aryl, carboxyaryl, dicarboxyaryl, heteroaryl, cycloalkyl, heterocycloalkyl, alkyloxy, alkylmercapto, aryloxy, arylmercapto, hydroxy, amino, nitro or cyano radicals or a solubilising or ionisable or ionised substituent such as -SO₃⁻, -PO₃²⁻, -CO₂⁻, *tert-*ammonium, cyclodextrin or sugar,
Y is a halogen atom such as fluorine, chlorine or bromine, or a substituted phenoxy or a substituted arylmercapto (phenylsulfanyl) function,
Kat is a particular number of Na⁺, K⁺, Ca²⁺, ammonium or other cations which are necessary for balancing the negative charge specified by the cyanine I and/or II,
n in cyanine I may have a value from 0 to 3, and
R₃ and R₄ in II are identical or different and may each be an aliphatic (preferably CH₃-) or heteroaliphatic group or may together form the divalent structural element -(CH₂)ₘ-, -(CH₂)ₘO(CH₂)ₘ-, -(CH₂)ₘS(CH₂)ₘ-, -(CH₂)ₘCH=CH- or -OCH=CH- and m and m' may be identical or different and may have a value from 2 to 6.

2. Hydrophilic markers according to claim 1, **characterised in that** the ω-carboxyalkyl function in the 3-position on the indole ring is in activated form, for example in NHS-ester, acid fluoride, TFP ester or STP ester form.

3. Hydrophilic markers according to claim 1, **characterised in that** the w-carboxyalkyl function in the 3-position on the indole ring is used, by way of an acid amide function, to activate the respective cyanines in the form of maleimides, halo-acetamides, hydrazides, amine-amidites or phosphoramidites.

4. Hydrophilic markers according to claims 1 and 3, **characterised in that** instead of the diastereoisomeric mixtures the corresponding pure diastereoisomers are used for labelling biomolecules.

5. Hydrophilic markers according to claims 1 to 4, **characterised in that** they are used for labelling biomolecules.

6. Use of the hydrophilic markers according to claims 1 to 4 as cyanines for covalent binding of amino acids, proteins, antibodies, nucleic acids, oligomers, DNA, RNA, biological cells, lipids, haptens, polymers, pharmaceuticals or polymer particles and use of the cyanine conjugates in optical, especially optical fluorescent, qualitative and quantitative determination processes including immune tests, hybridisation procedures, FRET systems, chromatographic or electrophoretic procedures and high throughput screening.

## Revendications

1. Marqueurs hydrophiles à base d'un mélange de cyanines diastéréomériques de structure générale I ou II 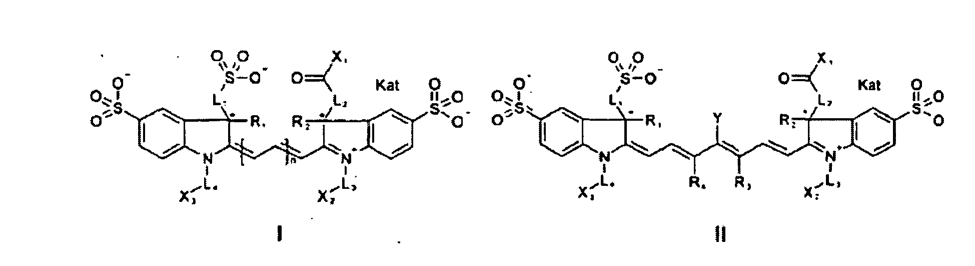 dans laquelle
R₁ et R₂, identiques ou différents, peuvent être respectivement, un groupe aliphatique (de préférence, CH₃-) ou hétéroaliphatique,
L₁ à L₄, identiques ou différents, désignent respectivement un groupe alkylène divalent linéaire (-(CH₂)ₒ- où o = 1 à 15), ramifié ou cyclique, qui peut être substitué par un ou plusieurs hétéroatomes tels que l'oxygène, l'azote substitué ou le soufre,
X₁ désigne les groupes -OH, -SH, NH₂, -NH-NH₂, -F, -CI, -Br, -I, -O-NHS (hydroxysuccinimidyl/hydroxysulfo-succinimidyl), -O-STP (4-sulfo-2,3,5,6-tétrafluorophénoxy), -O-TFP (2,3,5,6-tétrafluorophénoxy), benzotriazol ou -NR-L-OH, -NR-L-O-phosphoramidite, -NR-L-SH, -NR-L-CO-NH₂, -NR-L-CO-NH-NH₂, -NR-L-CHO, -NR-L-COOH, -NR-L-CO-ONHS, -NR-L-CO-O-STP, -NR-L-CO-O-TFP, -NR-L-NH-CO-CH₂-I et -NR-L-maléimide, où L est un groupe alkylène linéaire divalent (-(CH₂)_{o',} où o' = 1 à 15), ramifié ou cyclique qui peut être substitué par un ou plusieurs hétéroatomes tels que oxygène, azote substitué ou soufre et R peut être un atome d'hydrogène ou un radical R₁ ou R₂,
X₂ et X₃, identiques ou différents, peuvent être un atome d'hydrogène, un radical alkyle, tert-alkyle, aryle, carboxyaryle, dicarboxyaryle, hétéroaryle, cycloalkyle, hétérocycloalkyle, alkyloxy, alkylmercapto, aryloxy, arylmercapto, hydroxy, amino, nitro, cyano ou des substituants solubilisants ou ionisables ou ionisés tels que -SO₃⁻, -PO₃²⁻_{,} -CO₂⁻, tert-ammonium, cyclodextrine ou un sucre,
Y désigne un atome d'halogène tel qu'un atome de fluor, de chlore ou de brome, une fonction phénoxy substituée ou arylmercapto-(phényl-sulfanyle) substituée,
Kat désigne un nombre déterminé de Na⁺, K⁺, Ca²⁺, d'ammonium ou autres cations, qui sont nécessaires pour compenser la charge négative implicite induite par la cyanine I ou II,
la valeur de 0 à 3 peut être attribuée à n dans le groupe cyanine I et
R₃ et R₄ dans la structure II, identiques ou différents, peuvent représenter respectivement un groupe aliphatique (de préférence CH₃-) ou hétéroaliphatique ou forment conjointement, l'élément structurel divalent - (CH₂)ₘ-, -(CH₂)ₘO(CH₂)ₘ-, -(CH₂)ₘS(CH₂)ₘ-, -(CH₂)ₘCH=CH- ou -OCH=CH- et m ou m', identiques ou différents peuvent représenter les valeurs de 2 à 6.

2. Marqueurs hydrophiles selon la revendication 1, **caractérisé en ce que** la fonction ω-carboxyalkyle en position 3 sur le cycle indole se présente sous forme activée, par exemple sous forme de NHS-ester, fluorure acide, TFP ou STP-ester.

3. Marqueurs hydrophiles selon la revendication 1, **caractérisé en ce que** l'on utilise la fonction ω-carboxyalkyle en position 3 sur le cycle indole, pour activer par le biais d'une fonction amide acide, la cyanine respective en maléimide, halogénacétamide, hydrazide, amine ou phosphoramidite.

4. Marqueurs hydrophiles selon les revendications 1 et 3, **caractérisé en ce que** l'on utilise, à la place des mélanges de diastéréomères, les diastéréomères purs correspondants pour marquer des biomolécules.

5. Marqueurs hydrophiles selon les revendications 1 à 4, **caractérisé en ce que** l'on utilise ceux-ci pour le marquage de biomolécules.

6. Utilisation des marqueurs hydrophiles selon les revendications 1 à 4 comme cyanines pour la liaison covalente d'acides aminés, de protéines, d'anticorps, d'acides nucléiques, d'oligomères, d'ADN, d'ARN, de cellules biologiques, de lipides, d'haptènes, de polymères, de médicaments ou de particules de polymère et emploi des conjugués de cyanine dans des procédés de détermination qualitatifs et quantitatifs optiques, en particulier, optiques fluorescents comprenant des tests immunologiques, des procédés d'hybridation, des systèmes FRET, des procédés chromatographiques ou électrophorétiques et de détection à rendement élevé.
